# EUROPEAN PATENT APPLICATION

(11) **EP 2 659 856 A1**
(43) Date of publication of application: **06.11.2013**
(21) Application number: 13165137.4
(22) Date of filing: 24.04.2013
(51) Int. Cl.: A61B 19/00, A61B 6/00, H02G 11/02, A61B 18/22

(54) **Arrangement for supporting one or more cables, wires or hoses**

(30) Priority: 30.04.2012 US 201213459910
(71) Applicant: ELEKTA AB (publ.), 103 93 Stockholm (SE)
(72) Inventor: Nyman, Markus, SE-115 58 Stockholm (SE); Eldered, Kjell, SE-134 65 Ingarö (SE)
(74) Representative: Loqvist, Gabriel Mathias

(57) **Abstract**

The present invention relates to an arrangement for supporting one or more cables, wires or hoses during repeated movements between a first and second end position. The arrangement comprises: a component support structure; a component turnable between the first and second end position; one or more elongated cables, wires or hoses; and a winding element. The one or more cables, wires or hoses are lead along the support structure to the winding element and continues to component and the surplus length of the cables, wires or hoses absorbed within the winding element. The invention furthermore relates to a medical device comprising said arrangement.

## Description

### Field of the invention

The present invention relates to an arrangement for supporting one or more cables, wires or hoses during repeated movements between a first and second end position and a medical device comprising said arrangement.

### Background of the invention

Within many different technical areas, such as for example industrial robots or complex medical equipment, different components of the robot or equipment are changing position in relation to each other during use. In order to make it possible to access cables, wires or hoses between the different components of the robot or equipment in a reliable way, an arrangement that supports and adapts to the change in position between the different components is required. The cables, wires or hoses are for example used for power supply and/or communication between the different components.

The need for an arrangement that supports the cables, wires or hoses frequently occurs in complex structures comprising a number of different components that during use of the product are moving in relation to each other. One example of such a product is disclosed in US2011/0150186 where the disclosed radiotherapy installation comprises a treatment head that is both moving longitudinally along a support structure and turning around a substantially horizontal axis 14y. The movable treatment head requires power as well as connecting wires that makes it possible to send information between the treatment head and further components connected to the treatment head.

The described movement between the different components requires that the cables and/or wires have the desired length to allow movements to the most extended positions. However, the cables/wires must be supported when the treatment head is retracted to both support the cables during the movements and avoid that the surplus length of the cables/wires are damaged due to the repeated deformations of the cables/wires during use of the product.

Furthermore, in order to adapt to the movements, the cables and/or wires are sometimes bent in different directions which over time is devastating for the cables, wires or hoses since the risk for cable or wire breakdown increases with the number of deformations of the cable, wire or hose. This problem is of major concern for cables, wires or hoses with high stiffness since the high stiffness increases the risk for breakage because of the repeated deformations of the cable, wire or hose.

There is consequently a need for an improved arrangement for supporting cables/wires extending between components that during use are moving in relation to each other in order to reduce the risk for break downs of the cables, wires or hoses.

### Summary of the invention

The present invention, defined in the appended claims, provides an arrangement for supporting cables, wires or hoses that fulfils the needs defined above, and a medical device comprising said arrangement.

The claimed arrangement for supporting one or more cables, wires or hoses during repeated movements between a first and second end position, comprises:
a component support structure, said support structure is extending in a first axial direction and having a component support end;
a component turnably secured in the component support end of the support structure, said component is turnable between the first and second end position by rotation around a substantially horizontal axis arranged substantially perpendicular to said first axial direction;
one or more elongated cables, wires or hoses; and
a winding element arranged along the horizontal axis and comprising a hub element, two side walls extending radially outwards from the hub element defining a space for the one or more cables, wires or hoses between the side walls, an inlet opening and an exit opening;
wherein said one or more cables, wires or hoses are lead along the support structure to the space defined between the side walls of the winding element via the inlet opening, said one or more cables, wires or hoses extend from the inlet opening towards the hub, continues around the hub and from the hub via the exit opening in the winding element to the component, and said exit opening is arranged to rotate together with the component around the horizontal axis.

The claimed arrangement fulfils the needs defined above since the surplus length of the cable, wire or hose that result from the retraction of the component from the most extracted end position is absorbed within the winding element. This is achieved by an increase of the winding diameter of the cable, wire or hose winded on the hub element. In other words, the cable, wire or hose that in the most extracted end position is winded around the hub element is released and the diameter of the winded cable, wire or hose increased slightly to absorb the surplus length of the cable, wire or hose. This is a considerable advantage since the surplus length is absorbed completely within the winding element with only limited deformations within the different segments of the cables, wires or hoses. Furthermore, the deformations to absorb the surplus length of the cable, wire or hose is distributed over a considerable length of the cable, wire or hose which result in that each of the cable, wire or hose segments are exposed to a limited deformation. The length of the cable, wire or hose arranged within could also be adapted to adjust the deformation that each of the segments of the cable, wire or hose is exposed to by changing the dimensions of the winding element.

The invention has further advantages. First, the arrangement has a limited length in the direction of the rotational axis and requires a limited space since only the cable winding element has to be added to the product or structure where the arrangement for support of a surplus length of a cable, wire or hose is desired. Secondly, since the exit opening of the cable winding element is arranged to rotate together with the component, no further additional components except the winding element are required for achieving the desired support of the cable, wire or hose.

In one embodiment of the arrangement according to the invention, the one or more cables, wires or hoses extend at least one lap around the hub element when the component is arranged in the first end position and more than one lap when the component is arranged in the second end position. This embodiment is advantageous since the specified length and extension of the cables, wires or hoses ensures that the desired limited deformation of each cable, wire or hose segment is achieved.

In one embodiment of the arrangement, the cable winding element comprises a flexible dividing element that extend in substantially radial direction from the hub and divides the space between the two side walls into two compartments, and said one or more cables, wires or hoses enters the winding element in one of the compartments, extend around the hub element and continues into the second compartment via a passage in the flexible dividing element to avoid contact between adjacent laps of the one or more cables, wires or hoses, and exits the winding element via the exit opening. This embodiment is favourable since the flexible dividing element ensures that the different laps of the cables, wires or hoses are able to move radially inwards and outwards without interfering with each other and the surplus length absorbed in the best way possible.

In one embodiment of the arrangement, the first and second end positions of the component are arranged an angle α from each other, and said angle α is within the range of 180° to 350°. This embodiment is favourable since the specified range of the angle provides a very reliably support for the cables, wires or hoses in all position within the specified range.

In one embodiment of the arrangement, the winding element has an outer periphery and the inlet and exit openings of the cable winding element are arranged in said outer periphery. This embodiment of the winding element is favourable since the cables, wires or hoses could be lead from the component support structure to the winding element and further to the component without any sharp bends.

In one embodiment of the arrangement, the hub element has a cylindrical cross section in the plane transverse to said horizontal rotational axis. The cylindrical cross section of the hub is favourable since the risk for damages to the cables, wires or hoses winded around the circular periphery of the hub is reduced.

In one embodiment of the arrangement, the hub has an axial length within the range of the double and triple diameter of the largest one of the one or more elongated cables, wires or hoses. This embodiment is favourable since the specified axial length of the hub element ensures that two laps of cables, wires or hoses could be arranged side by side around the hub which ensures that the desired change of the cable, wire or hose diameter and absorption of the surplus length of cable, wire or hose take place in an effective way.

In one embodiment of the arrangement, the winding element is divided in a first and a second winding element segment, said first segment comprising the inlet opening, the side wall arranged adjacent to the component support structure and a first hub element segment adjacent to said side wall, and said second winding element segment comprising the exit opening, a second hub element segment and the remaining side wall, and wherein said first winding element segment is stationary arranged together with the component support structure while the second winding element segment is arranged to rotate together with the component around the horizontal rotational axis. This embodiment is favourable since the cables, wires or hoses enters the stationary arranged first segment of the winding element before it continues to the second segment of the cable winding element that is rotating together with the component. This means that the risk for wear between the cable, wire or hose and the inlet opening or the side wall arranged adjacent to the component support structure is eliminated and the entire surplus length of the cable, wire or hose is taken care of within the winding element.

In one embodiment of the arrangement, the flexible dividing element is arranged between the first and second winding element segment. This position of the flexible dividing element further improves the performance of the winding element since the movements of the cables, wires or hoses in radial direction is guided by the dividing element and the risk for wear between adjacent components reduced.

In one embodiment of the arrangement, the one or more elongated cables, wires or hoses, after they have exited the cable winding element, extend in a direction substantially parallel to the horizontal axis towards the component.

In one embodiment of the arrangement, the at least one of the one or more cables, wires or hoses has a diameter exceeding 10 mm. The arrangement is particularly suitable for cables, wires or hoses with diameters exceeding 10 mm since these cables, wires or hoses, due to their large diameter, have a high stiffness that increases the risk for damages when exposes to repeted deformations.

The invention furthermore relates to a medical device for treatment of a patient, said medical device comprising a support frame and a device body from which cables, wire or hoses extend to a component that is movable between a first and second end position. The cables, wires or hoses are supported by an arrangement according to anyone of the embodiments disclosed above.

The different described embodiments of the arrangement could of course be combined in different ways without departing from the scope of the invention that will be described more in detail in the detailed description.

### Brief description of the drawings

One embodiment of the arrangement according to the invention is illustrated in the appended figures.
- Figure 1: illustrates a schematic perspective view of the arrangement.
- Figure 2a to c: discloses the arrangment and the cables, wires or hoses of the arrangement in selected positions.
- Figure 3: illustrates a medical devic comprising the arrangement according to the invention schematically.

### Detailed description of embodiments

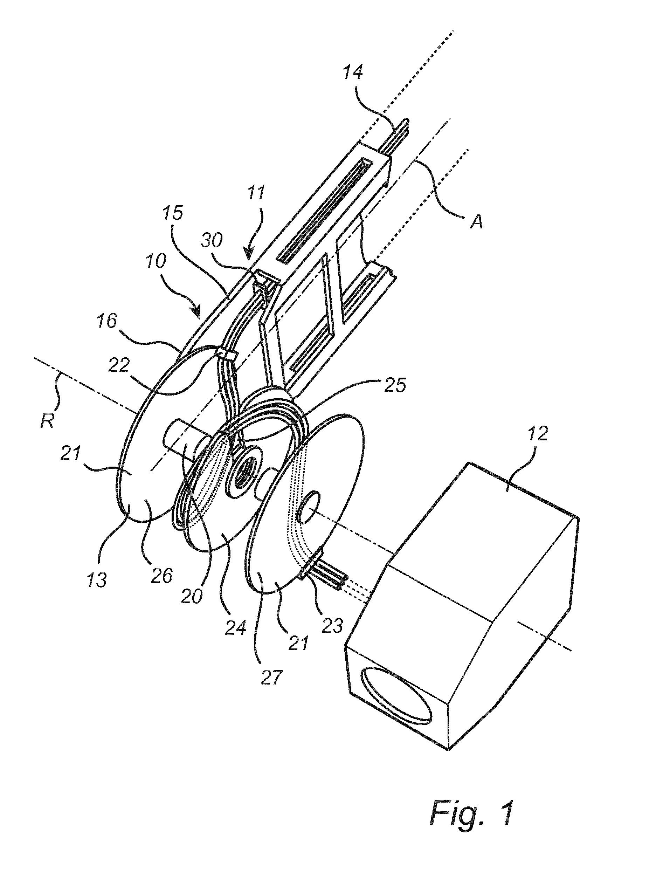

In figure 1, a perspective view of an arrangement 10 according to the invention is illustrated schematically. The arrangement comprises a component support structure 11 for a component 12 rotatably secured in the support structure 11, a winding element 13 and three cables 14 extending from the component support structure 11 to the winding element 13 before they continue to the component that is turnable between a first P1 and a second end P2 position by rotation around a substantially horizontal rotational axis R.

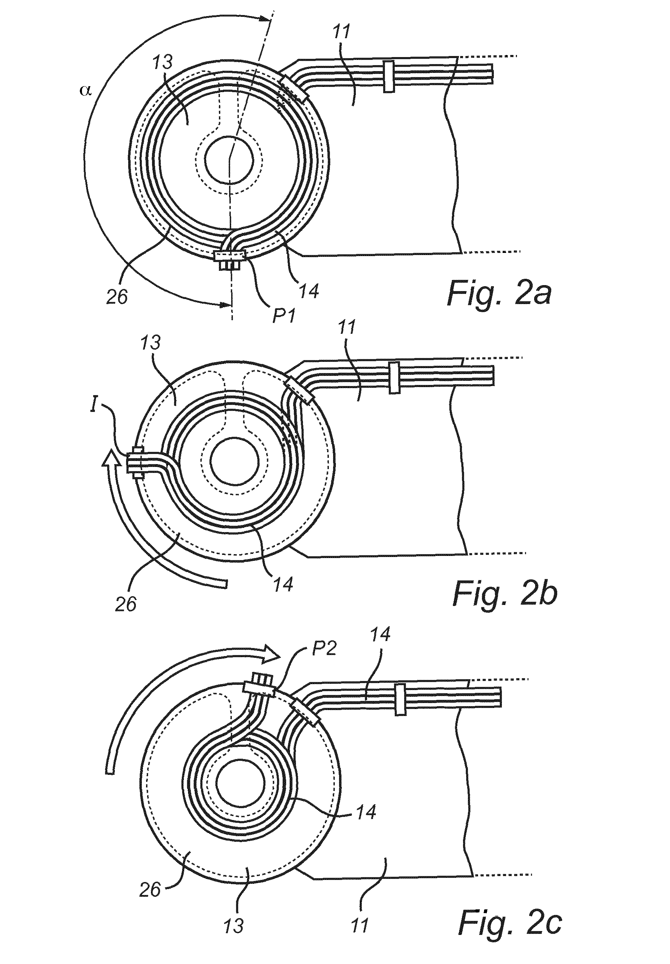

In figure 1 and 2, three cables 14 extending side by side are illustrated. The number of cables is however irrelevant. The arrangement according to the invention could be used for any number of cables, wires or hoses, alternatively combinations of these as long as the dimensions of the winding element 13 are adapted accordingly.

The component support structure 11 is in the illustrated embodiment formed as an elongated arm 15 extending in a longitudinal direction A. The arm 15 is formed as a frame work in order to achieve the desired structural strength and stiffness, and make room for a number of different components directed to different functions of the product or equipment in which the inventive arrangement is arranged, not illustrated in the figures. These components could be arranged within, or on the outside surface of the arm 15 as long as they not interfere with other components or parts of the structure during use of the product or equipment. Examples of additional components arranged within, or on, the component support structure are components for turning the component 12 around the rotational axis and means for detecting the angular position of the component 12 around the rotational axis R.

The elongated component support structure 11 comprises a component support end 16 in which the component 12 is arranged. The component 12 is rotatably secured in the support structure 11 and arranged to rotate around the rotational axis R arranged substantially perpendicular to the longitudinal axis A of the component support structure 11. The rotational axis R is substantially horizontal in order to ensure the desired function of the arrangement 10. Bearings ensure that the component 12 could be turned easily between the first P1 and the second end position P2. The end positions P1, P2 of the component and the winding element are illustrated in figure 2a and 2c. Other well known solutions could however also be used for securing the component 12 in the support structure 11 as long as the desired turnable securing of the component 12 is achieved.

The component 12 is preferably secured in the support structure 11 via one or more bearings to ensure that the component 12 is turnable even though considerable loads could be present in the area of the securing. Depending on the type of structure or equipment the arrangement 10 is used in, the specific type of component 12 arranged in the component support end 16 of the component support structure 11 could be for example a robot arm, a laser, an X-ray device etc. These components requires power in order to work as intended and/or connections for communication and information transfer between the component 12 and corresponding equipment arranged separated from the component 12 are required. The power is supplied, either in electric, hydraulic or pneumatic form and a number of cables, wires or hoses must consequently be lead between the component support structure 11 and the component 12.

The winding element 13 is arranged adjacent to the component 12 in order to support the cables 14 extending between the component support structure 11 and the component 12. The winding element 13 is either arranged on the same side of the component support structure 11 as the component 12 as illustrated in figure 1, alternatively on the opposite side of the component support structure.

In figure 1 the winding element 13 and the component 12 are arranged on the same side of the support structure 11. However, arranging the winding element and the component on opposite sides of the support structure could be advantageous since the weight distribution is improved and both the component and the winding element are easily accessible for maintenance and repair.

The winding element 13 comprises a hub element 20, preferably with a circular cross-section, arranged coaxially with the rotational axis R, and circular disk-shaped wall elements 21 arranged in each axial end of the hub element. The wall elements 21 are extending in substantially radial direction from each axial end of the hub element 20 in order to form an annular space between the wall elements 21 for the cables 14. The axial length of the hub element and diameter of the wall elements are adapted to the number and dimensions of the cables, wires or hoses that need to be supported and absorbed within the winding element 13. Preferably the axial length of the hub element 20 is at least twice the diameter of the cable, wire or hoses, alternatively twice the diameter of the set of cables, wires or hoses extending between the support structure and the component. The winding element 13 furthermore comprises an inlet opening 22 and an exit opening 23 for the cables. The dimensions of the inlet 22 and exit 23 opening are adapted to the dimensions and number of cables, wires or hoses passing through the openings. The winding element 13 furthermore comprises a dividing element 24 made of a flexible material. The dividing element has the shape of a C with a passage 25 for the cables 14 formed between the two ends of the C-shaped dividing element 24. The dividing element 24 is arranged transverse to the rotational axis R with the passage 25 facing upwards between the two wall elements 21 in order to divide the space within the winding element into two smaller compartments.

The winding element 13, illustrated in figure 1, is divided in a first 26 and a second 27 winding element segment. The winding element is divided close to the axial centre of the hub element 20 transverse to the rotational axis R. The first winding element segment 26 comprises the inlet opening 22, arranged in the outer periphery of the winding element, the side wall 21 arranged adjacent to the component support structure 11 and a first hub element segment from which the first wall element extend. The second winding element segment 27 comprises the outlet opening 23, the second wall element 21 arranged in the opposite end of the hub element 20 as the first wall element and a second hub element segment from which the second wall element extend.

The first winding element segment 26 is secured to the support structure 11 and prevented from turning together with the component while the second winding element segment 27 is structurally connected with the component 12, for example by an elongated rotational shaft, in order to always turn in accordance with the component. The flexible dividing element 24 is arranged in the area between the first 26 and the second 27 winding element segment to form the two compartments. The flexible dividing element 24 is stationary arranged together with the first winding element segment 26.

The cables 14 are extending along the support structure 11 towards the winding element 13. The cables are secured in the support structure 11 by fasteners 30 to remain in the intended position on the support structure 11. The cables14 enters the stationary arranged first segment 26 of the cable winding element 13 via the inlet opening 22 positioned in the outer periphery of the winding element 13 and arranged in a position facing the position of the cables 14on the support structure 11. The cables 14 extend from the inlet opening 22 around the hub element 20 within the first winding element segment 26, i.e. the compartment arranged adjacent to the first wall element 21 and the support structure 11. The cables 14extend about one lap around the hub element 20 before they are lead via the passage 25 in the flexible dividing element 24 into the second compartment on the opposite side of the dividing element 24. The cables14 continues within the second compartment and exits the winding element via the exit opening 23 arranged in the outer periphery of the winding element 13 close to the second wall element 21. The cables 14 continue from the exit opening 23 to the component 12.

In figure 2a to 2c three different positions of the winding element of the arrangement are illustrated in order to better disclose the advantages of the cable support arrangement 10 according to the invention. In order for the component 12 to be turnable between the first end position P1, illustrated in figure 2a, and the second end position P2 illustrated in figure 2c, each of the cables 14 must have a length that permit the component 12 to turn to the most extracted position, the second end position P2 in which the cables 14 are winded almost two laps around the hub element 20.

However, in order to prevent damages to the cables 14 and/or surrounding components the surplus length of the cables 14 is absorbed within the winding element 13. This is achieved by an increase in the diameter of the winded laps of cables 14 within the winding element 13. This is illustrated both in figure 2b that is illustrating an intermediate position I of the component and the second winding element segment 26, and figure 2a that discloses the first end position P1 of the component and the second winding element segment 26. In order to further improve the arrangement and ensure that the entire surplus length of the cables stay within the winding element, the cables could be secured in axial direction in the exit opening 23.

The first P1 and second end position P2 are arranged an angle α from each other. In figure 2a to 2c this angle is about 200°. Preferably, the angle α is within the range of 150° to 350°.

The arrangement according to the invention eliminates wear between the cables, wires or hoses and the inlet opening and the side wall arranged adjacent to the component support structure since all movements of the cables take place within the winding element. The movement and wear between adjacent laps of the cables within the winding element is limited by the dividing element that prevents contact between the adjacent laps and the deformation of the cable segments limited since very limited deformation of the cables take place during the change in winding diameter of the cables.

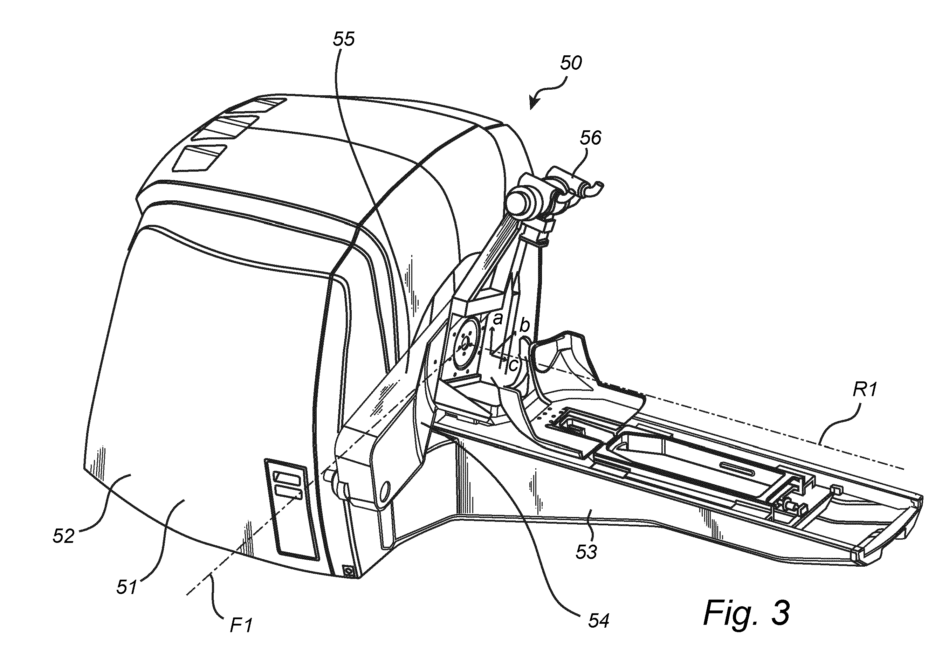

In figure 3, the arrangement according to the invention is used in a device 50 for medical treatment of a patient. The medical device 50 comprises a rigid support frame 51 intended to be arranged on the floor within a hospital, and a device body 52 in which a number of different components for operating and controlling the medical device are arranged. Adjacent to the frame and the device body a treatment bed 53 for positioning of the patient in the correct position during treatment is illustrated. The design of the medical device could however be modified in many different ways.

The medical device furthermore comprises an arrangement according to any one of the embodiments previously described, i.e. a movable component support structure 54 extending in a first axial direction from the support frame 51. The support structure 54 is movable between an upright storage position and a substantially horizontal position. In the outer end 55 of the support structure 54 a component 56 is turnably secured in the support structure54. The component 56 is for example a robot arm, a laser or an X-ray device depending on the intended use of the medical device 50. The component 56 is turnable between a first and second end position by rotation around a substantially horizontal rotational axis R1 arranged substantially perpendicular to said first axial direction F1. In order to power and communicate with the component 56 one or more elongated cables, wires or hoses are extending from said device body to the component via a winding element arranged along the horizontal rotational axis on the side of the support structure facing the device body 52. The winding element and the cables, wires or hoses are not visible in figure 3. The winding element, more extensively described above with reference to figure 1 and 2, comprises a hub element, two side walls extending radially outwards from the hub element defining a space for the one or more cables, wires or hoses between the side walls, an inlet opening and an exit opening.

The one or more cables, wires or hoses are lead from the device body 52 along the support structure 54 to the space defined between the side walls of the winding element via the inlet opening, said one or more cables, wires or hoses extend from the inlet opening towards the hub, continues around the hub and from the hub via the exit opening in the winding element to the component, and said exit opening is arranged to rotate together with the component 56 around the horizontal rotational axis in order to provide the desired support and absorption of the surplus length of the cables, wires or hoses during use of the medical device.

The different embodiments described above could all be combined and modified in different ways without departing from the scope of the invention that is defined by the appended claims.

## Claims

1. Arrangement for supporting one or more cables, wires or hoses during repeated movements between a first and second end position, said support arrangement comprising:
a component support structure, said support structure is extending in a first axial direction and having a component support end;
a component turnably secured in the component support end of the support structure, said component is turnable between the first and second end position by rotation around a substantially horizontal rotational axis arranged substantially perpendicular to said first axial direction;
one or more elongated cables, wires or hoses; and
a winding element arranged along the horizontal rotational axis and comprising a hub element, two side walls extending radially outwards from the hub element defining a space for the one or more cables, wires or hoses between the side walls, an inlet opening and an exit opening;
wherein said one or more cables, wires or hoses are lead along the support structure to the space defined between the side walls of the winding element via the inlet opening, said one or more cables, wires or hoses extend from the inlet opening towards the hub, continues around the hub and from the hub via the exit opening in the winding element to the component, and said exit opening is arranged to rotate together with the component around the horizontal rotational axis.

2. Arrangement according to claim 1, wherein the one or more cables, wires or hoses extend at least one lap around the hub element when the component is arranged in the first end position, and more than one lap when the component is arranged in the second end position.

3. Arrangement according to claim 1 or 2, wherein the cable winding element comprises a flexible dividing element that extend in substantially radial direction from the hub and divides the space between the two side walls into two compartments, and said one or more cables, wires or hoses enters the cable winding device in one of the compartments, extend around the hub element and continues into the second compartment via a passage in the flexible dividing element to avoid contact between adjacent laps of the one or more cables or wires, and exits the winding element via the exit opening.

4. Arrangement according to anyone of the previous claims, wherein the first and second end position of the component are arranged an angle α from each other, and said angle α is within the range of 180° to 350°.

5. Arrangement according to anyone of the previous claims, wherein the winding element has an outer periphery and the inlet and exit openings of the cable winding element are arranged in said outer periphery.

6. Arrangement according to anyone of the previous claims, wherein the hub element has a cylindrical cross section in the plane transverse to said horizontal axis.

7. Arrangement according to anyone of the previous claims, wherein the hub element has an axial length between the double and triple diameter of the largest one of the one or more elongated cables, wires or hoses.

8. Arrangement according to anyone of the previous claims, wherein the winding element is divided in a first and a second cable winding element segment, said first segment comprising the inlet opening, the side wall arranged adjacent to the component support structure and a first hub element segment adjacent to said side wall, and said second segment comprising the exit opening, a second hub element segment and the remaining side wall, and wherein said first cable winding element segment is stationary arranged together with the component support structure while the second winding element segment is arranged to rotate together with the component around the horizontal axis.

9. Arrangement according to claim 8, wherein the flexible dividing element is
arranged between the first and second cable winding element segment.

10. Arrangement according to anyone of the previous claims, wherein the one
or more elongated cables, wires or hoses, after they have exited the winding element, extend in a direction substantially parallel to the first horizontal axis towards the component.

11. Arrangement according to anyone of the previous claims, wherein at least
one of the one or more cables, wires or hoses has a diameter exceeding 10 mm.

12. Medical device for treatment of a patient, said medical device comprising a support frame and a device body;
a movable component support structure extending in a first axial direction from the support frame;
a component turnably secured in the support structure, said component is turnable between a first and second end position by rotation around a substantially horizontal rotational axis arranged substantially perpendicular to said first axial direction;
one or more elongated cables, wires or hoses extending from said device body to the component; and
a winding element arranged along the horizontal rotational axis and comprising a hub element, two side walls extending radially outwards from the hub element defining a space for the one or more cables, wires or hoses between the side walls, an inlet opening and an exit opening;
wherein said one or more cables, wires or hoses are lead from the device body along the support structure to the space defined between the side walls of the winding element via the inlet opening, said one or more cables, wires or hoses extend from the inlet opening towards the hub, continues around the hub and from the hub via the exit opening in the winding element to the component, and said exit opening is arranged to rotate together with the component around the horizontal rotational axis.

13. Medical device according to claim 12, wherein the component is a robot arm, a laser or an X-ray device.
